# EUROPÄISCHE PATENTSCHRIFT

(11) **EP 1 917 253 B1**
(45) Veröffentlichungstag und Bekanntmachung des Hinweises auf die Patenterteilung: **07.01.2015**
(21) Anmeldenummer: 06778214.4
(22) Anmeldetag: 10.08.2006
(51) Int. Cl.: C07D 265/36, C07C 205/45, C07C 211/45, C07C 233/18, C07C 233/13

(54) **VERFAHREN ZUR HERSTELLUNG VON BETAMIMETIKA**
METHOD FOR PRODUCING BETAMIMETICS
PROCEDE DE PRODUCTION DE BETAMIMETIQUES

(30) Priorität: 15.08.2005 EP 05107470
(43) Veröffentlichungstag der Anmeldung: 07.05.2008
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim am Rhein (DE)
(72) Erfinder: KRUEGER, Thomas, 88353 Kisslegg (DE); RIES, Uwe, 88400 Biberach (DE); SCHNAUBELT, Juergen, 88447 Oberhoefen/Warthausen (DE); RALL, Werner, 88441 Mittelbiberach (DE); LEUTER, Zeno A., 88250 Weingarten (DE); DURAN, Adil, 88400 Biberach (DE); SOYKA, Rainer, 88400 Biberach (DE)
(74) Vertreter: Simon, Elke Anna Maria
(86) Internationale Anmeldenummer: PCT/EP2006/065217
(87) Internationale Veröffentlichungsnummer: WO 2007/020227

(56) Entgegenhaltungen:
- WO-A-2004/087142
- WO-A-2005/111005
- DE-B- 1 144 713
- US-A- 4 460 581
- US-A- 4 656 168
- N.P.BUU-HOÏ, C.T.LONG, N.D.XUONG: "alpha,alpha-Dimethyl-beta-arylethylamines , and Their Behavior in the Bischler-Napieralski Reaction" JOURNAL OF ORGANIC CHEMISTRY, Bd. 23, Nr. 1, 1958, Seiten 42-45, XP002406161
- T.YAMASHITA: "Redox-Photosensitized Aminations of 1,2-Benzo-1,3-cycloalkadienes, Arylcyclopropanes, and Quadricyclane with Ammonia" JOURNAL OF ORGANIC CHEMISTRY, Bd. 68, Nr. 20, 2003, Seiten 7618-7624, XP002406162
- M.JULIA ET AL.: "A BIOMIMETIC SYNTHESIS OF CHRYSANTHEMOL" TETRAHEDRON, Bd. 37, Nr. 2, 1981, Seiten 325-332, XP002406163
- J W TIMBERLAKE ET AL: "Thiadiaziridine 1,1-Dioxides: Synthesis and Chemistry" JOURNAL OF ORGANIC CHEMISTRY, Bd. 46, Nr. 10, 1981, Seiten 2082-2089, XP002406285

## Beschreibung

Die vorliegende Erfindung betrifft ein Verfahren zur Herstellung von einem Betamimetikum der Formel 1

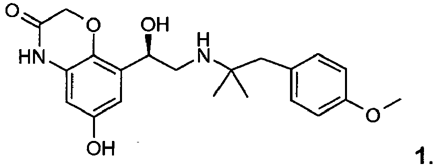

### HINTERGRUND DER ERFINDUNG

Betamimetika (ß-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei. Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung ein Verfahren zur Herstellung von Betamimetika bereitzustellen, die einerseits bei der Therapie der COPD oder Asthma einen therapeutischen Nutzen entfalten und darüber hinaus durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines zur Therapie von COPD oder Asthma einmal täglich applizierbaren Arzneimittels eingesetzt werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β₂-Adrenozeptor gekennzeichnet sind.

### DETAILLIERTE BESCHREIBUNG DER ERFINDUNG

Die vorliegende Erfindung betrifft ein Verfahren zur Herstellung einer Verbindung der der Formel 1, 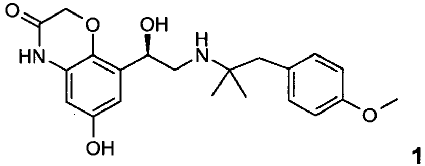 dadurch gekennzeichnet, dass eine Verbindung der Formel 2b, 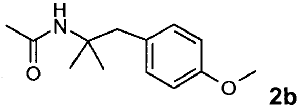 zu einer Verbindung der Formel 1 b, 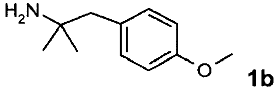 umgesetzt wird und anschließend mit einer Verbindung der Formel **1a,** 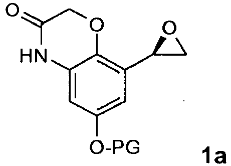 worin PG für eine Schutzgruppe steht, in einem organischen Lösungsmittel zu einer Verbindung der Formel **1c**, 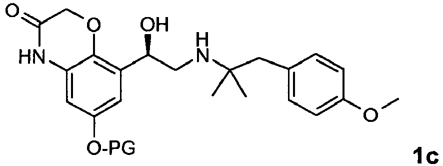 worin PG die oben genannte Bedeutung hat, umgesetzt und aus dieser durch Abspaltung der Schutzgruppe PG die Verbindung der Formel 1 erhalten wird.

Im erfindungsgemäßen Verfahren wird eine Verbindung der Formel **1a** in einem geeigneten Lösungsmittel mit einer Verbindung der Formel 1 b umgesetzt. Als geeignete Lösungsmittel kommen organische Lösungsmittel in Betracht, besonders bevorzugte Lösungsmittel sind ausgewählt aus der Gruppe bestehend aus Tetrahydrofuran, Toluol, Ethanol, *n*-Propanol, *n*-Butanol, *n*-Butylacetat, Dimethylformamid, Methoxyethanol, Ethylenglykol und Dioxan. Erfindungsgemäß besonders bevorzugt gelangen als Lösungsmittel zum Einsatz *n*-Propanol, Tetrahydrofuran und Dioxan, wobei Dioxan und *n-*Propanol besondere Bedeutung zukommt.

Bezogen auf eingesetzte Verbindung **1a** gelangen erfindungsgemäß bevorzugt wenigstens stöchiometrische Mengen an Verbindung **1b** zum Einsatz. Gegebenenfalls kann Verbindung **1b** auch im Überschuss, beispielsweise in bis zu 3 Äquivalenten, vorzugsweise in bis zu 2,5 Äquivalenten, besonders bevorzugt in etwa 1 bis 2, gegebenenfalls in 1 bis 1.5 Äquivalenten bezogen auf eingesetzte Verbindung **1a** eingesetzt werden.

Die Umsetzung erfolgt vorzugsweise bei erhöhter Temperatur, vorzugsweise bei einer Temperatur von über 40°C, besonders bevorzugt bei einer Temperatur von über 50°C. Besonders bevorzugt wird Reaktionsmischung bis zur Siedetemperatur des eingesetzten Lösungsmittels erhitzt.

Bei dieser Temperatur wird die Umsetzung sodann in einem Zeitraum von etwa 1 bis 72 Stunden, vorzugsweise in 10 bis 60 Stunden, besonders bevorzugt in 20 bis 50 Stunden durchgeführt.

Nach vollendeter Reaktion wird das Lösungsmittel entfernt und der verbleibende Rückstand in einem organischen, polaren Lösungsmittel, bevorzugt einem C₁₋₈-Alkohol oder C₃₋₈-Ester, besonders bevorzugt in Ethanol oder Ethylacetat, aufgenommen und filtriert. Das Filtrat wird angesäuert, bevorzugt mit einer Mineralsäure, besonders bevorzugt mit Salzsäure und nach einer Dauer von etwa 10 Minuten bis 12 Stunden, vorzugsweise 20 Minuten bis 6 Stunden, besonders bevorzugt 30 Minuten bis 3 Stunden wird das Produkt abfiltriert.

Die Abspaltung der Schutzgruppe PG aus Verbindungen der Formel **1a** erfolgt bevorzugt mittels Hydrierung in einem geeigneten Lösungsmittel. Als geeignete Lösungsmittel kommen organische Lösungsmittel, bevorzugt organische, polare Lösungsmittel in Betracht, besonders bevorzugte Lösungsmittel sind ausgewählt aus der Gruppe bestehend aus Tetrahydrofuran, verschiedene C₃₋₈-Ester und C₁₋₈-Alkohole. Erfindungsgemäß bevorzugt gelangen als Lösungsmittel Tetrahydrofuran, Ethanol und Methanol zum Einsatz, wobei Ethanol und Methanol besondere Bedeutung zukommt.

Zur Hydrierung im erfindungsgemäßen Verfahren werden bevorzugt Katalysatoren in Gegenwart von Wasserstoff eingesetzt. Bevorzugte Katalysatoren sind geeignete Übergangsmetallkatalysatoren, bevorzugt heterogene Übergangsmetallkatalysatoren, besonders bevorzugt Palladium-haltige Katalysatoren, insbesondere ein Palladium-Kohle-Gemisch.

Die Hydrierung erfolgt bevorzugt in Gegenwart eines Überschuss von Wasserstoff. Dieser wird erfindungsgemäß durch einen Wasserstoffdruck von 1 bar bis 10 bar, bevorzugt zwischen 2 und 7 bar, besonders bevorzugt zwischen 2,5 und 4,5 bar, bereitgestellt.

Bevorzugt wird die Hydrierung bei erhöhter Temperatur, bevorzugt von 25 bis 70°C, besonders bevorzugt von 30 bis 60°C, insbesondere von 35 bis 50°C durchgeführt. Nach vollendeter Reaktion wird der Katalysator, bevorzugt durch Filtration, entfernt.

Danach wird das Lösungsmittel entfernt und das Produkt aus einem geeigneten organischen Lösungsmittel, bevorzugt einem C₁₋₈-Alkohol oder einem Gemisch aus C₁₋₈-Alkoholen, besonders bevorzugt aus einem Gemisch aus Methanol und einem Alkohol ausgewählt aus der Gruppe bestehend aus *i*-Propanol, *n*-Propanol und Ethanol umkristallisiert.

Erfindungsgemäß bevorzugt ist ein Verfahren, worin die Verbindung der Formel **1a** durch Umsetzung einer Verbindung der Formel **2a,** 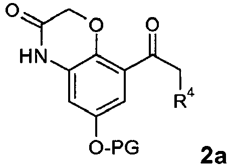 worin PG die in Anspruch 1 genannte Bedeutung hat und R⁴ Halogen, bevorzugt Brom oder Chlor, bedeutet, hergestellt wird.

Im erfindungsgemäßen Verfahren wird eine Verbindung der Formel **2a** in einem geeigneten Lösungsmittel mit DIP-Chlorid (Diisopinocampheylchloroboran) umgesetzt. Als geeignete Lösungsmittel kommen bevorzugt organische Lösungsmittel in Betracht. Bevorzugte Lösungsmittel sind ausgewählt aus der Gruppe bestehend aus Diethylether, *tert*-Butyl-methylether 2-Methyltetrahydrofuran, Tetrahydrofuran, Toluol und Dioxan. Erfindungsgemäß besonders bevorzugt gelangen als Lösungsmittel zum Einsatz *tert-*Butyl-methylether, Tetrahydrofuran und Dioxan, wobei Dioxan und Tetrahydrofuran besondere Bedeutung zukommt.

Das DIP-Chlorid kann in Reinform oder in Form einer Lösung, bevorzugt in einem inerten, organischen Lösungsmittel, besonders bevorzugt einem aliphatischen Lösungsmittel, insbesondere Pentan, Hexan, Heptan oder Octan, eingesetzt werden.

Die Zugabe des DIP-Chlorids erfolgt bei verminderter Temperatur im Reaktionsmedium, bevorzugt liegt die Temperatur dabei unter 0°C, besonders bevorzugt unter -10°C, insbesondere erfolgt die Zugabe bei -20 bis -40°C.

Die Zugabe des DIP-Chlorids erfolgt über einen Zeitraum von 10 min bis 6 Stunden, bevorzugt von 30 min bis 4 Stunden, besonders bevorzugt von 1 bis 3 Stunden. Insbesondere erfolgt die Zugabe in einem Zeitraum von 70 bis 110 min.

Bezogen auf eingesetzte Verbindung **2a** gelangen erfindungsgemäß bevorzugt wenigstens stöchiometrische Mengen an DIP-Chlorid zum Einsatz. Gegebenenfalls kann das DIP-Chlorid auch im Überschuss, beispielsweise in bis zu 3 Äquivalenten, vorzugsweise bis zu 2,5 Äquivalente, besonders bevorzugt in etwa 1,5 bis 2,5 Äquivalenten bezogen auf eingesetzte Verbindung **2a** eingesetzt werden.

Nach erfolgter Zugabe des DIP-Chlorids wird das Reaktionsgemisch über einen Zeitraum von 10 min bis 4 Stunden, bevorzugt von 30 min bis 3 Stunden, besonders bevorzugt von 40 bis 80 min, insbesondere wird das Reaktionsgemisch nach erfolgter Zugabe noch für Zeitraum von 50 bis 70 min gerührt. Während diesem Zeitraum wird das Reaktionsgemisch auf eine Temperatur von -20 bis 20°C, besonders bevorzugt von -10 bis 10°C, insbesondere von -5 bis 5°C, eingestellt.

Ist die gewünschte Temperatur erreicht, wird, bezogen auf die eingesetzte Menge an DIP-Chlorid, eine wenigstens stöchiometrische Menge an Natriumhydroxid (NaOH), in Wasser gelöst, zugegeben. Gegebenenfalls kann das NaOH auch im Überschuss, beispielsweise in bis zu 3 Äquivalenten, vorzugsweise in bis zu 2,5 Äquivalenten, besonders bevorzugt in etwa 1,5 bis 2,5 Äquivalenten, bezogen auf die eingesetzte Menge DIP-Chlorid, eingesetzt werden. Bevorzugt wird in der Reaktionsmischung nach erfolgter Zugabe von NaOH ein pH-Wert von 12 bis 14, besonders bevorzugt von 12,5 bis 13,5, insbesondere von 12,7 bis 13,3, gemessen.

Nachdem der gewünschte pH-Wert eingestellt wurde, wird das Reaktionsgemisch über einen Zeitraum von 10 min bis 4 Stunden, bevorzugt von 30 min bis 3 Stunden, besonders bevorzugt von 40-80 min, insbesondere wird das Reaktionsgemisch noch für Zeitraum von 50-70 min gerührt. Während diesem Zeitraum wird das Reaktionsgemisch auf eine Temperatur von 0 bis 40°C, besonders bevorzugt von 10 bis 30°C, insbesondere von 15 bis 25°C, eingestellt. Anschließend wird das Reaktionsgemisch mit einer Säure, bevorzugt einer Mineralsäure, besonders bevorzugt mit Salzsäure, auf einen pH-Wert von 7 bis 10, besonders bevorzugt von 8 bis 9, insbesondere von 8,2 bis 8,8, eingestellt.

Abschließend kann das Produkt durch Extraktion mit einem organischen Lösungsmittel aus dem Reaktionsgemisch isoliert werden und durch Fällung mit einem weiteren geeigneten organischen Lösungsmittel als Feststoff erhalten werden.

Erfindungsgemäß bevorzugt ist ein Verfahren, worin die Verbindung der Formel **2a** durch Umsetzung einer Verbindung der Formel **3a**, 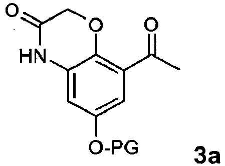 worin PG die in Anspruch 1 genannte Bedeutung hat, hergestellt wird.

Im erfindungsgemäßen Verfahren wird eine Verbindung der Formel **3a** in einem geeigneten Lösungsmittel mit einem Halogenierungsreagenz umgesetzt. Als geeignete Lösungsmittel kommen organische Lösungsmittel zum Einsatz. Bevorzugte Lösungsmittel sind ausgewählt aus der Gruppe bestehend aus Essigsäure, Butylacetat, Methylenchlorid, Tetrahydrofuran, Toluol und Dioxan. Erfindungsgemäß besonders bevorzugt sind die Lösungsmittel Tetrahydrofuran und Dioxan.

Als Halogenierungsreagenz kommt in einer bevorzugten Ausführungsform der Erfindung ein Bromierungsreagenz, besonders bevorzugt Brom, *N*-Bromsuccinimid, Benzyltrimethylammoniumtribromid und Tetrabutylammoniumtribromid, zur Verwendung. Bezogen auf eingesetzte Verbindung **3a** gelangen erfindungsgemäß bevorzugt wenigstens stöchiometrische Mengen an Halogenierungsreagenz zum Einsatz. Gegebenenfalls kann das Halogenierungsreagenz auch im Überschuss, beispielsweise in bis zu 3 Äquivalenten, vorzugsweise in bis zu 2 Äquivalenten, besonders bevorzugt in etwa 1 bis 1,5 Äquivalenten, bezogen auf eingesetzte Verbindung **3a**, eingesetzt werden. Das Halogierungsreagenz kann dabei in einem Lösungsmittel, bevorzugt in einem organischen, polaren Lösungsmittel, besonders bevorzugt in Methanol, Ethanol und Dioxan, insbesondere in Methanol und Dioxan, oder in einem Gemisch davon, insbesondere in einem Gemisch von Methanol und Dioxan, zu dem Reaktionsgemisch zugegeben werden.

Die Umsetzung erfolgt vorzugsweise bei einer Temperatur von 0 bis 40°C, bevorzugt bei einer Temperatur von 10 bis 30°C, besonders bevorzugt bei einer Temperatur von 15 bis 25°C.

Nach erfolgter Zugabe des Halogenierungsreagenzes wird das Reaktionsgemisch über einen Zeitraum von 10 min bis 6 Stunden, bevorzugt von 30 min bis 4 Stunden, besonders bevorzugt von 90 bis 150 min, gerührt.

Zur Isolierung des Produktes wird dem Reaktionsgemisch Wasser zugegeben, wobei das Gemisch auf eine Temperatur von -10°C bis 10°C, bevorzugt von 0 bis 10°C, besonders bevorzugt von 0 bis 5°C abgekühlt und für einen Zeitraum von 10 min bis 4 Stunden, bevorzugt von 30 min bis 2 Stunden, besonders bevorzugt von 50 bis 70 min, nach erfolgter Zugabe des Wassers gerührt wird. Das Produkt kann nach Filtration oder Zentrifugation und Trocknung erhalten werden.

Erfindungsgemäß bevorzugt ist ein Verfahren, worin die Verbindung der Formel **3a** durch Umsetzung einer Verbindung der Formel **4a,** 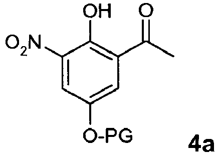 worin PG die in Anspruch 1 genannte Bedeutung hat, hergestellt wird.

Im erfindungsgemäßen Verfahren wird eine Verbindung der Formel **4a** in einem geeigneten Lösungsmittel hydriert. Als geeignete Lösungsmittel kommen organische Lösungsmittel, bevorzugt organische, polare Lösungsmittel in Betracht. Besonders bevorzugte Lösungsmittel sind ausgewählt aus der Gruppe bestehend aus Dimethylformamid, *N*-Methylpyrrolidinon, Tetrahydrofuran, 2-Methyltetrahydrofuran, Toluol und Dioxan. Erfindungsgemäß sind als Lösungsmittel besonders bevorzugt: Dimethylformamid, Tetrahydrofuran, 2-Methyltetrahydrofuran und Dioxan, wobei Dimethylformamid und 2-Methyltetrahydrofuran eine besondere Bedeutung zukommt.

Zur Hydrierung im erfindungsgemäßen Verfahren werden bevorzugt Katalysatoren in Gegenwart von Wasserstoff eingesetzt. Bevorzugte Katalysatoren sind geeignete Übergangsmetallkatalysatoren, bevorzugt heterogene Übergangsmetallkatalysatoren, besonders bevorzugt nickelhaltige oder platinhaltige Katalysatoren, insbesondere Platinoxid.

Die Hydrierung erfolgt bevorzugt in Gegenwart eines Überschusses von Wasserstoff. Dieser wird erfindungsgemäß durch einen Wasserstoffdruck von 1 bar bis 10 bar, bevorzugt von 2 bis 7 bar, besonders bevorzugt von 2,5 bis 4,5 bar, bereitgestellt.

Bevorzugt wird die Hydrierung bei einer Temperatur von 0 bis 50°C, bevorzugt von 10 bis 40°C, insbesondere von 20 bis 30°C, durchgeführt. Nach vollendeter Reaktion wird der Katalysator, bevorzugt durch Filtration, von der flüssigen Phase entfernt.

Das in der Lösung befindliche Zwischenprodukt **4a^{#},** 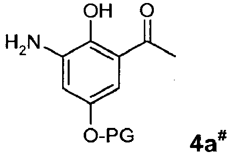 worin PG die in Anspruch 1 genannte Bedeutung hat, kann isoliert oder direkt weiter zu einer Verbindung der Formel **3a** umgesetzt werden.

Dem erfindungsgemäßen Verfahren entsprechend wird eine Base, bevorzugt eine schwache Base, besonders bevorzugt ein Carbonat, insbesondere Kaliumcarbonat, vorgelegt und die Verbindung der Formel **4a**^{#}, in Reinform oder in einer Lösung, insbesondere in Form der im vorangegangenen Schritt vom Hydrierungskatalysator abfiltrierten Lösung, zugegeben.

Bezogen auf die eingesetzte Verbindung **4a** gelangen erfindungsgemäß bevorzugt wenigstens doppeltstöchiometrische Mengen der Base zum Einsatz. Gegebenenfalls kann die Base auch im Überschuss, beispielsweise in bis zu 6 Äquivalenten, vorzugsweise in bis zu 4 Äquivalenten, besonders bevorzugt in etwa 3 bis 3,5 Äquivalenten, bezogen auf eingesetzte Verbindung **4a,** eingesetzt werden.

Anschließend wird dem Reaktionsgemisch Chloracetylchlorid zugeführt. Die Zugabe des Chloracetylchlorids erfolgt über einen Zeitraum von 10 min bis 2 Stunden, bevorzugt von 15 min bis 1 Stunde, besonders bevorzugt von 25 bis 35 min.

Bezogen auf eingesetzte Verbindung **4a** gelangen erfindungsgemäß bevorzugt wenigstens stöchiometrische Mengen des Chloracetylchlorids zum Einsatz. Gegebenenfalls kann das Chloracetylchlorid auch im Überschuss, beispielsweise in bis zu 4 Äquivalenten, vorzugsweise in bis zu 3 Äquivalenten, besonders bevorzugt in etwa 1,5 bis 2 Äquivalenten, bezogen auf eingesetzte Verbindung **4a,** eingesetzt werden.

Nach erfolgter Zugabe des Chloracetylchlorids wird das Reaktionsgemisch über einen Zeitraum von 10 min bis 6 Stunden, bevorzugt von 1 bis 4 Stunden, besonders bevorzugt von 140 bis 160 min, gerührt.

Die Umsetzung erfolgt vorzugsweise bei erhöhter Temperatur, vorzugsweise bei einer Temperatur von über 40°C, besonders bevorzugt bei einer Temperatur von über 50°C, besonders bevorzugt von 60°C bis 70°C.

Die Reaktion wird durch Zugabe von Wasser beendet. Die Verbindung der Formel **3a** kann durch Extraktion des Reaktionsgemisches mit Wasser und anschließendem Umkristallisieren aus einem geeigneten organischen Lösungsmittel gereinigt und isoliert werden. Für die Kristallisation eignet sich bevorzugt ein aliphatischer Kohlenwasserstoff, besonders bevorzugt ein aliphatischer, cyclischer Kohlenwasserstoff, insbesondere Cyclohexan und Methylcyclohexan.

Erfindungsgemäß bevorzugt ist ein Verfahren, worin die Verbindung der Formel 4a durch Umsetzung einer Verbindung der Formel **5a**, 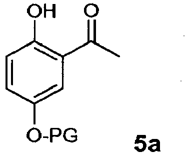 worin PG die in Anspruch 1 genannte Bedeutung hat, hergestellt wird.

Im erfindungsgemäßen Verfahren wird eine Verbindung der Formel **5a** in einem geeigneten Lösungsmittel mit einem Nitrierungsreagenz umgesetzt. Als geeignete Lösungsmittel kommen organische Lösungsmittel und Säuren, bevorzugt organische, protische Lösungsmittel und Säuren in Betracht. Besonders bevorzugte Lösungsmittel sind Essigsäure und Schwefelsäure, insbesondere Essigsäure.

Zur Nitrierung im erfindungsgemäßen Verfahren werden bevorzugt 6-65%-ige Salpetersäure, sowie Nitroniumtetraflouroborat oder Acetylnitrat eingesetzt. Besonders bevorzugt ist Salpetersäure, insbesondere 65%-ige Salpetersäure.

Bezogen auf eingesetzte Verbindung **5a** gelangen erfindungsgemäß bevorzugt wenigstens stöchiometrische Mengen des Nitrierungsreagenz zum Einsatz. Gegebenenfalls kann das Nitrierungsreagenz auch im Überschuss, beispielsweise in bis zu 2 Äquivalenten, vorzugsweise in bis zu 1,5 Äquivalenten, besonders bevorzugt in etwa 1 bis 1,1 Äquivalenten, bezogen auf eingesetzte Verbindung **5a**, eingesetzt werden.

Nach erfolgter Zugabe des Nitrierungsreagenz wird das Reaktionsgemisch über einen Zeitraum von 10 min bis 4 Stunden, bevorzugt von 20 min bis 3 Stunden, besonders bevorzugt von 40 bis 80 Minuten gerührt.

Anschließend wird das Reaktionsgemisch mit so viel Wasser verdünnt, dass die Verbindung der Formel **4a** aus der Lösung ausfällt. Zur Vervollständigung der Kristallisation wird noch für 20 min bis 3 Stunden, bevorzugt 30 min bis 2 Stunden, besonders bevorzugt 40-80 min, bei einer Temperatur von 0°C bis 20°C, bevorzugt bei 5°C bis 15°C, besonders bevorzugt bei 8°C bis 12°C, gerührt. Die Verbindung der Formel **4a** kann durch Abtrennung von der flüssigen Phase, bevorzugt durch Filtration oder Zentrifugation, erhalten werden.

Erfindungsgemäß bevorzugt ist ein Verfahren, worin die Verbindung der Formel **5a** durch Umsetzung einer Verbindung der Formel **6a,** 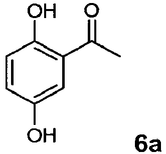 hergestellt wird.

Im erfindungsgemäßen Verfahren wird eine Verbindung der Formel **6a** in einem geeigneten Lösungsmittel mit einer Schutzgruppe PG-A, worin A eine geeignete Abgangsgruppe wie beispielsweise Chlor, Brom, Iod, Methansulfonyl, Trifluormethansulfonyl oder *p*-Toluolsulfonyl bedeutet, umgesetzt. Bevorzugt wird eine Schutzgruppe verwendet, die sich, wie unter der Abspaltung der Schutzgruppe PG aus Verbindungen der Formel **1a** beschrieben, entfernen lässt. Besonders bevorzugt wird eine gegebenenfalls substituierte Benzyl-Schutzgruppe verwendet.

Erfindungsgemäß ist ein Verfahren, worin die Verbindung der Formel 1 b durch Umsetzung einer Verbindung der Formel 2b, 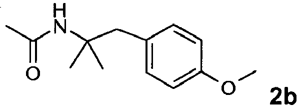 hergestellt wird.

Im erfindungsgemäßen Verfahren wird eine Verbindung der Formel 2b in einem geeigneten Lösungsmittel mit einer starken Base umgesetzt. Als geeignete Lösungsmittel kommen organische Lösungsmittel in Betracht, besonders bevorzugte Lösungsmittel sind ausgewählt aus der Gruppe bestehend aus Ethanol, 2-Ethoxyethanol und Ethylenglykol oder Gemische davon. Erfindungsgemäß besonders bevorzugt gelangen 2-Ethoxyethanol oder Ethylenglykol oder ein Gemisch davon als Lösungsmittel zum Einsatz. Bevorzugt besteht das Gemisch aus gleichen Volumenteilen 2-Ethoxyethanol und Ethylenglykol, wobei auch ein geringer Überschuss des einen oder des anderen Lösungsmittel möglich ist.

Als starke Base finden insbesondere anorganische Hydroxide, bevorzugt Erdalkali- oder Alkalihydroxide, insbesondere Natriumhydroxid und Kaliumhydroxid, Verwendung. Erfindungsgemäß kommt Kaliumhydroxid besondere Bedeutung zu.

Bezogen auf eingesetzte Verbindung 2b gelangen erfindungsgemäß bevorzugt wenigstens stöchiometrische Mengen der starken Base zum Einsatz. Gegebenenfalls kann die starke Base auch im Überschuss, beispielsweise in bis zu 8 Äquivalenten, vorzugsweise in bis zu 6 Äquivalenten, bevorzugt in etwa 2 bis 4, besonders bevorzugt in 3,5 bis 4,5 Äquivalenten, bezogen auf eingesetzte Verbindung 2b, eingesetzt werden.

Die Umsetzung erfolgt vorzugsweise bei erhöhter Temperatur, vorzugsweise bei einer Temperatur von über 100°C, besonders bevorzugt bei einer Temperatur von über 120°C. Besonders bevorzugt wird die Reaktionsmischung auf 140-160°C, insbesondere auf 145-155°C, erhitzt.

Anschließend wird das Reaktionsgemisch zur Extraktion mit einem Lösungsmittel und Wasser verdünnt. Als Lösungsmittel kommt Toluol, Xylol, Heptan, Methylcyclohexan oder *tert*-Butyl-methylether, bevorzugt Toluol oder Xylol, besondere Bedeutung zu. Die wässrige Phase wird entfernt, die organische Phase in weiteren Reinigungsschritten mit Wasser extrahiert. Das Wasser kann dabei durch gängige Zusatzstoffe sauer, neutral oder alkalisch sein. Bevorzugt wird die organische Phase einmal mit angesäuertem Wasser und anschließend mit basischem Wasser extrahiert. Das Produkt kann aus der organischen Phase durch Entfernung des Lösungsmittels gewonnen werden.

Erfindungsgemäß bevorzugt ist ein Verfahren, worin die Verbindung der Formel 2b durch Umsetzung einer Verbindung der Formel **3b,** 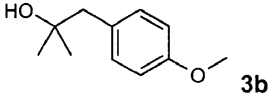 hergestellt wird.

Im erfindungsgemäßen Verfahren wird eine Verbindung der Formel 3b in einem geeigneten Lösungsmittel mit Acetonitril in Gegenwart einer Säure umgesetzt. Als geeignete Lösungsmittel kommen Säuren, bevorzugt organische Säuren in Betracht, besonders bevorzugtes Lösungsmittel ist Essigsäure.

Bezogen auf eingesetzte Verbindung **3b** gelangen erfindungsgemäß bevorzugt wenigstens stöchiometrische Mengen von Acetonitril zum Einsatz. Bevorzugt wird das Acetonitril im Überschuss, beispielsweise in bis zu 6 Äquivalenten, vorzugsweise in bis zu 5 Äquivalenten, besonders bevorzugt in etwa 2 bis 4 Äquivalenten, insbesondere in 2,5 bis 3,5 Äquivalenten, bezogen auf eingesetzte Verbindung 3b, eingesetzt.

Als Säure, in deren Gegenwart die Reaktion durchgeführt wird, wird bevorzugt Schwefelsäure, Ameisensäure, p-Toluolsulfonsäure, Methansulfonsäure, Perchlorsäure, oder Polyphosphorsäure, besonders bevorzugt Schwefelsäure, verwendet.

Bezogen auf eingesetzte Verbindung **3b** gelangen erfindungsgemäß bevorzugt wenigstens stöchiometrische Mengen der Säure zum Einsatz. Gegebenenfalls kann die Säure auch im Überschuss, beispielsweise in bis zu 2 Äquivalenten, vorzugsweise in bis zu 1,5 Äquivalenten, besonders bevorzugt in etwa 1 bis 1,1 Äquivalenten, bezogen auf eingesetzte Verbindung **5a,** eingesetzt werden. Nach erfolgter Zugabe der Säure wird das Reaktionsgemisch über einen Zeitraum von 1 bis 5 Stunden, bevorzugt von 2 bis 4 Stunden, besonders bevorzugt von 170 bis 190 min, gerührt.

Die Umsetzung erfolgt vorzugsweise bei erhöhter Temperatur, vorzugsweise bei einer Temperatur von über 30°C, besonders bevorzugt bei einer Temperatur von über 40°C, besonders bevorzugt von 45°C bis 60°C. Überrascherweise wurde gefunden, dass bei diesem Verfahren keine unerwünschte Spaltung der Methyletherfunktion auftritt, wie sie auf Grund der Literatur (Can. J. Chem. 56 (1978), 3054-3058) erwartet würde.

Anschließend wird das Reaktionsgemisch in einen zweiten Reaktor überführt, der ein gekühltes Lösungsmittelgemisch enthält. Als geeignete Lösungsmittel kommen Gemische von polaren und unpolaren Lösungsmitteln, bevorzugt wässrige, organische, polare und unpolare Lösungsmittel in Betracht. Besonders bevorzugte Lösungsmittel als Bestandteil des Gemisches sind ausgewählt aus der Gruppe bestehend aus Wasser, *tert*-Butyl-methylether, Tetrahydrofuran, Toluol, Dioxan, Hexan, Cyclohexan und Methylcyclohexan. Erfindungsgemäß besonders bevorzugt gelangen als Bestandteil des Gemisches, Wasser, *tert*-Butylmethylether, Tetrahydrofuran, Toluol, Cyclohexan und Methylcyclohexan zum Einsatz, wobei einem Gemisch aus Wasser, *tert*-Butylmethylether und Methylcyclohexan besondere Bedeutung zukommt.

Vorzugsweise wird das Gemisch der Lösungsmittel auf verminderter Temperatur, vorzugsweise bei einer Temperatur von unter 20°C, besonders bevorzugt bei einer Temperatur von unter 15°C, besonders bevorzugt von 0°C bis 15°C, gehalten.

Um das Produkt aus dem Lösungsmittel auszufällen wird der pH-Wert des Reaktionsgemisches erhöht, bevorzugt in den basischen Bereich, besonders bevorzugt von pH 8 bis 12, insbesondere von pH 9 bis 10. Bevorzugt wird zur Erhöhung des pH-Wertes eine Ammoniaklösung verwendet

Nach erfolgter Zugabe und Einstellung des pH-Wertes wird das Reaktionsgemisch über einen Zeitraum von 10 min bis 3 Stunden, bevorzugt von 20 min bis 2 Stunden, besonders bevorzugt von 50 bis 70 min, gerührt.

Anschließend wird das Produkt abzentrifugiert und mit den oben genannten zur Reaktion verwendeten Lösungsmitteln gewaschen. Durch weitere Umkristallisation, bzw. Fällung, z.B. mit C₁₋₈-Alkoholen und Wasser, kann ein Produkt höherer Reinheit erhalten werden.

Erfindungsgemäß bevorzugt ist ein Verfahren, worin die Verbindung der Formel 3b durch Umsetzung einer Verbindung der Formel **4b,** 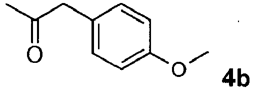 hergestellt wird.

Im erfindungsgemäßen Verfahren wird eine Verbindung der Formel 4b in einem geeigneten Lösungsmittel mit Methylmagnesiumbromid einer Grignard-Reaktion unterzogen. Als geeignete Lösungsmittel kommen organische Lösungsmittel zum Einsatz. Bevorzugte Lösungsmittel sind ausgewählt aus der Gruppe bestehend aus Diethylether, *tert*-Butyl-methylether, Tetrahydrofuran, Toluol und Dioxan. Erfindungsgemäß besonders bevorzugt gelangen *tert-*Butyl-methylether, Tetrahydrofuran und Toluol als Lösungsmittel zum Einsatz.

Die Umsetzung erfolgt bevorzugt bei Raumtemperatur, vorzugsweise bei einer Temperatur von 10 bis 20°C , besonders bevorzugt bei einer Temperatur von 15 bis 25°C.

Nach erfolgter Zusammenführung der Edukte wird das Reaktionsgemisch über einen Zeitraum von 10 min bis 3 Stunden, bevorzugt von 20 min bis 2 Stunden, besonders bevorzugt von 50 bis 70 min, gerührt.

Zum Beenden der Reaktion wird dem Reaktionsgemisch Wasser und eine Säure, bevorzugt Schwefelsäure, zugesetzt. Das Produkt kann nach Extraktion der organischen Phase mit Standardmethoden durch Abziehen des Lösungsmittels isoliert werden. Durch Umkristallisation aus einem organischen, unpolaren Lösungsmittel, bevorzugt *n*-Heptan, kann die Reinheit des Produktes erhöht werden.

### VERWENDETE BEGRIFFE UND DEFINTIONEN

Unter einem "organischen Lösungsmittel" wird im Rahmen der Erfindung ein organsicher, niedermolekularer Stoff verstanden, der andere organische Stoffe auf physikalischem Wege zur Lösung bringen kann. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Adsorption in der Originalgestalt wieder gewonnen werden können. Aus verschiedenen Gründen können nicht nur die reinen Lösungsmittel, sondern Gemische, die die Lösungseigenschaften vereinigen, verwendet werden. Beispielsweise seien genannt:
- Alkohole, bevorzugt Methanol, Ethanol, Propanol, Butanol, Octanol, Cyclohexanol;
- Glykole, bevorzugt Ethylenglykol, Diethylenglykol;
- Ether / Glykolether, bevorzugt Diethylether, *tert*-Butyl-methylether, Dibutylether, Anisol, Dioxan, Tetrahydrofuran, Mono-, Di-, Tri-, Polyethylenglykolether;
- Ketone, bevorzugt Aceton, Butanon, Cyclohexanon;
- Ester, bevorzugt Essigsäureester, Glykolester;
- Amide u.a. Stickstoff-Verbindungen, bevorzugt Dimethylformamid, Pyridin, N-Methylpyrrolidon, Acetonitril;
- Schwefel-Verbindungen, bevorzugt Schwefelkohlenstoff, Dimethylsulfoxid, Sulfolan;
- Nitro-Verbindungen, bevorzugt Nitrobenzol;
- Halogenkohlenwasserstoffe, bevorzugt Dichlormethan, Chloroform, Tetrachlormethan, Tri- und Tetrachlorethen, 1,2-Dichlorethan, Chlorfluorkohlenstoffe;
- aliphatische oder alicyclische Kohlenwasserstoffe, bevorzugt Benzine, Petrolether, Cyclohexan, Methylcyclohexan, Decalin, Terpen-L.; oder
- aromatische Kohlenwasserstoffe, bevorzugt Benzol, Toluol, o-Xylol, *m*-Xylol, *p*-Xylol; oder entsprechende Gemische davon.

Unter dem Begriff "C₁₋₈-Alkohol" werden verzweigte und unverzweigte Alkohole mit 1 bis 8 Kohlenstoffatomen verstanden und einer oder zwei Hydroxygruppen. Bevorzugt sind Alkohole mit 1 bis 4 Kohlenstoffatome. Beispielsweise werden hierfür genannt: Methanol, Ethanol, *n*-Propanol, *iso-*Propanol, *n*-Butanol, *iso*-Butanol, *sec*-Butanol oder *tert*-Butanol. Gegebenenfalls werden für vorstehend genannte Moleküle auch die Abkürzungen MeOH, EtOH, n-PrOH, *i*-PrOH, n-BuOH, *i*-BuOH, *t*-BuOH, etc. verwendet. Sofern nicht anders beschrieben, umfassen die Definitionen Propanol, Butanol, Pentanol und Hexanol alle denkbaren isomeren Formen der jeweiligen Reste. So umfasst beispielsweise Propanol *n-*Propanol und *iso*-Propanol, Butanol umfasst *iso*-Butanol, *sec*-Butanol und *tert*-Butanol etc.

Unter dem Begriff "C₃₋₈-Ester" werden verzweigte und unverzweigte Ester mit insgesamt 3 bis 8 Kohlenstoffatomen verstanden. Bevorzugt sind Ester der Essigsäure mit 3 bis 6 Kohlenstoffatome. Beispielsweise werden hierfür genannt: Methylacetat, Ethylacetat, *n*-Propylacetat, *i*-Propylacetat oder n-Butylacetat, bevorzugt ist Ethylacetat.

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

"Schutzgruppen" in Sinne der vorliegenden Erfindung sind als Sammelbezeichnung für solche organische Reste zu verstehen, mit denen bestimmte funktionelle Gruppen eines mehrere aktive Zentren enthaltenden Moleküls vorübergehend gegen den Angriff von Reagenzien geschützt werden können, so dass Reaktionen nur an den gewünschten (ungeschützten) Stellen stattfinden. Die Schutzgruppen sollen unter milden Bedingungen selektiv einzuführen sein. Sie müssen für die Dauer des Schutzes unter allen Bedingungen der durchzuführenden Reaktionen und Reinigungsoperationen stabil sein; Racemisierungen und Epimerisierungen müssen unterdrückt werden. Schutzgruppen sollen wieder unter milden Bedingungen selektiv und idealerweise mit hoher Ausbeute abspaltbar sein. Die Wahl einer geeigneten Schutzgruppe, die Bedingungen zur Umsetzung (Lösungsmittel, Temperatur, Dauer, etc.), aber auch die Möglichkeiten eine Schutzgruppe wieder zu entfernen sind im Stand der Technik bekannt (z.B. Philip Kocienski, Protecting Groups, 3rd ed. 2004, THIEME, Stuttgart, ISBN: 3131370033). Bevorzugte Schutzgruppen sind ggf. substituiertes Benzyl, Diphenylmethyl, Trityl, Tosyl, Mesyl oder Triflat, besonders bevorzugt ist ggf. substituiertes Benzyl. 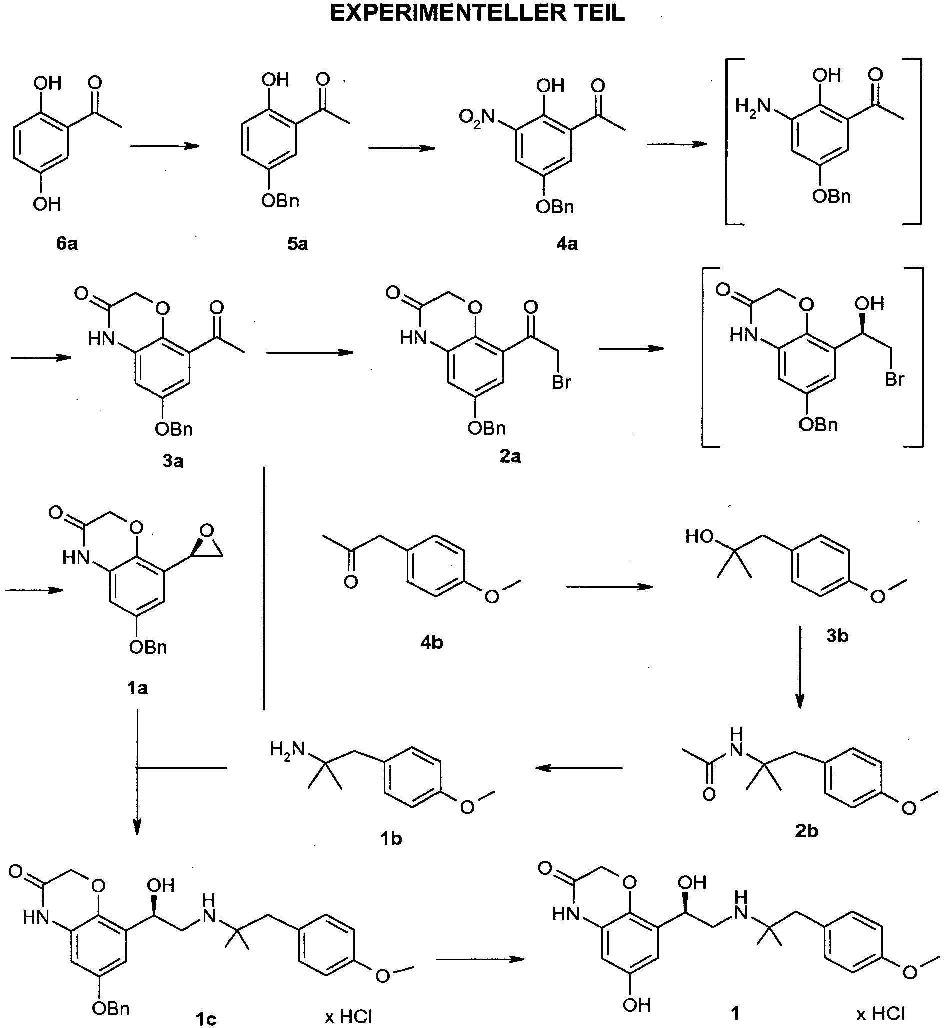 worin Bn die Bedeutung von Benzyl hat.
**8-[(1R)-1-Hydroxy-2-[[2-(4-methoxy-phenyl)-1,1-dimethyl-ethyl]-amino]ethyl]-6-(phenylmethoxy)-2H-1,4-benzoxazin-3(4H)-on-hydrochlorid** der Formel 1c: 7,00 kg (23,54 mol) 8-(2R)-Oxiranyl-6-(phenylmethoxy)-2H-1,4-benzoxazin-3(4H)-on 1a und 34,70 mol (4-methoxy-phenyl)-1,1-dimethyl-ethylamin der Formel 1b werden in 70 L 1,4-Dioxan vorlegt. Der Reaktorinhalt wird auf 97°C aufgeheizt und bei dieser Temperatur 48 Stunden nachgerührt. Anschließend wird auf 40°C abgekühlt und 56 L 1,4-Dioxan im Vakuum abdestilliert. Zum Rückstand werden 70 L Ethanol zugegeben, auf 25°C abkühlt, und bei 25°C innerhalb von 15 Minuten 4,15 kg (34,14 mol) Salzsäure (30%) zugegeben. Danach wird angeimpft und bis zur Kristallisation gerührt. Die entstandene Suspension wird auf 20°C abkühlt und 2 Stunden nachgerührt. Das Produkt wird zentrifugiert, mit 21 L Ethanol nachgewaschen und im Vakuum bei 50°C getrocknet. Ausbeute (1c): 84-90%, Enantiomerenreinheit laut HPLC: 89.5 -99,5%.

**6-Hydroxy-8-[(1R)-1-hydroxy-2-[[2-(4-methoxy-phenyl)-1,1-dimethyl-ethyl]-amino]ethyl]-2H-1,4-benzoxazin-3(4H)-on-hydrochlorid** der Formel 1: 19,49 mol 8-[(1R)-1-Hydroxy-2-[[2-(4-methoxy-phenyl)-1,1-dimethyl-ethyl]-amino]ethyl]-6-(phenylmethoxy)-2H-1,4-benzoxazin-3(4H)-on-hydrochlorid der Formel **1c** werden im Hydrierreaktor vorgelegt und mit 40 L Methanol suspendiert. 500 g Palladium auf Kohle 10% (50% Wasser) werden in 17 L Methanol suspendiert und in den Hydrierreaktor eingezogen. Es wird bei 40°C Innentemperatur und bei 3 bar Wasserstoffdruck hydriert, bis keine Wasserstoffaufnahme mehr zu verzeichnen ist. Der Katalysator wird abfiltriert und mit 13,3 L Methanol nachgespült. Es werden 60 L Methanol unter schwachem Vakuum abdestilliert. Wenn keine Kristallbildung erfolgt, wird der Destillationsrückstand angeimpft. Anschließend werden bei 50°C 30 L *i*-Propanol zudosiert und innerhalb von 1 Stunde auf 0°C abgekühlt. Bei 0°C wird 1 Stunde nachgerührt, abgesaugt und mit 15 L kaltem *i*-Propanol nachgewaschen. Das feuchte Produkt wird in 50 Liter Methanol gelöst. Die entstandene Lösung wird klarfiltriert, und das Druckfilter mit 10 Liter Methanol nachgespült. Anschließend werden unter schwachem Vakuum (ca. 500 mbar) 52 L Methanol abdestilliert. Wenn keine Kristallbildung erfolgt, wird der Destillationsrückstand angeimpft. Anschließend werden 22,6 L *i*-Propanol zudosiert. Es wird auf 0°C abgekühlt, und die Suspension wird 1 Stunde bei 0°C gerührt. Die Suspension wird abgesaugt, mit 15 Liter kaltem *i*-Propanol gewaschen und im Vakuum bei 50°C getrocknet. Ausbeute (1): 63-70%.

**1-[2-Hydroxy-5-(phenylmethoxy)-phenyl]-ethanon:** 20 kg (131,4 mol) 2-Acetylhydrochinon **6a** werden in 150 L Methylisobutylketon gelöst und mit 19,98 kg (144,6 mol) Kaliumcarbonat versetzt. Bei 60°C werden 22,48 kg (131,5 mol) Benzylbromid zugegeben. Die Reaktionsmischung wird 20 Stunden bei 60°C gerührt. Die Reaktionsmischung wird auf 25°C gekühlt und der Feststoff wird abfiltriert. Das Filtrat wird zweimal mit je einer Lösung aus 0,96 kg (11,8 mol) Natriumhydroxid-Lsg. (50%) und 60 L Wasser bei 25°C gewaschen. Das Methylisobutylketon wird im Vakuum weitgehend abdestilliert, und der Rückstand wird in 80 L Methanol bei 60°C gelöst. Die Lösung wird auf 0°C abgekühlt und 1 Stunde bei dieser Temperatur zur Vervollständigung der Kristallisation gerührt. Ausbeute **(5a):** 24,07 kg (75,6%), Chemische Reinheit laut HPLC: 99,2%.

**1-[2-Hydroxy-3-nitro-5-(phenylmethoxy)-phenyl]-ethanon:** 10,00 kg (41,27 mol) 1-[2-Hydroxy-5-(phenylmethoxy)-phenyl]-ethanon **5a** werden in 50 L Essigsäure gelöst. Zu dieser Lösung werden bei 15 bis 20°C 4,40 kg (45,40 mol) Salpetersäure 65% zudosiert. Das Zulaufgefäß wird mit 4 L Essigsäure nachgespült. Das Reaktionsgemisch wird 1 Stunde nachgerührt. Nach Animpfen wird mit 50 L Wasser versetzt. Die erhaltene Suspension wird zur Vervollständigung der Kristallisation 1 Stunde bei 10°C gerührt. Das Produkt wird zentrifugiert und bei 50°C getrocknet. Ausbeute **(4a):** 10,34 kg (87,2%), Chemische Reinheit laut HPLC: 99,0%.

**8-Acetyl-6-(phenylmethoxy)-2*H*-1,4-benzoxazin-3(4*H*)-on:** 15,00 kg (52,22 mol) 1-[2-Hydroxy-3-nitro-5-(phenylmethoxy)-phenyl]-ethanon **4a,** 0,165 kg Platin(IV)oxid und 45 L 2-Methyltetrahydrofuran werden bei 3 bar Wasserstoffdruck und einer Innentemperatur von 25°C hydriert, bis keine Wasserstoffaufnahme mehr zu verzeichnen ist. Der Katalysator wird abfiltriert und mit 20 L 2-Methyltetrahydrofuran gewaschen. In einem weiteren Reaktor werden 23,09 kg (167,09 mol) Kaliumcarbonat vorgelegt, und das Reaktionsgemisch aus dem ersten Reaktor wird zugegeben. Es wird mit 22 L 2-Methyltetrahydrofuran nachgespült. Anschließend werden innerhalb von 30 Minuten 9,44 kg (83,55 mol) Chloracetylchlorid zur Suspension zudosiert. Nach 2,5 Stunden Reaktionszeit bei 65°C werden 101 L Wasser zugegeben. Die wässrige Phase wird bei 55°C abgetrennt. Anschließend werden aus der organischen Phase 34 L 2-Methyltetrahydrofuran im Vakuum abdestilliert. Nach Aufheizen auf Rückflusstemperatur werden innerhalb von 30 Minuten 180 L Methylcyclohexan unter Rückfluss zudosiert. Die erhaltene Suspension wird auf 20°C abgekühlt und zur Vervollständigung der Kristallisation noch 1 Stunde bei dieser Temperatur nachgerührt. Anschließend wird der Niederschlag abzentrifugiert, mit 113 L Methylcyclohexan gewaschen und bei 50°C getrocknet. Ausbeute **(3a):** 12,70 kg (81,8%), Chemische Reinheit laut HPLC: 98,4%.

**8-(Bromacetyl)-6-(phenylmethoxy)-2*H*-1,4-benzoxazin-3(4*H*)-on:** 12,00 kg (40,36 mol) 8-Acetyl-6-(phenylmethoxy)-2*H*-1,4-benzoxazin-3(4*H*)-on **3a** werden in 108 L 1,4-Dioxan gelöst. Anschließend wird eine Lösung aus 24,33 kg (50,45 mol) Tetrabutylammoniumtribromid in 48 L 1,4-Dioxan und 12 L Methanol bei 20°C zur Suspension zudosiert. Der Reaktorinhalt wird 2 Stunden bei 20°C gerührt. Anschließend werden 72 L Wasser bei 20°C innerhalb von 15 Minuten zugegeben. Nach Abkühlung auf 3°C wird 1 Stunde gerührt, zentrifugiert und mit einer Mischung aus 9 L 1,4-Dioxan und 4,5 L Wasser gewaschen. Anschließend wird mit 60 L Wasser gewaschen und im Vakuum bei 50°C getrocknet. Ausbeute **(2a):** 11,29 kg (74,4%), Chemische Reinheit laut HPLC: 98,0%.

**8-(2*R*)-Oxiranyl-6-(phenylmethoxy)-2*H*-1,4-benzoxazin-3(4*H*)-on:** 12,00 kg (31,90 mol) 8-(Bromacetyl)-6-(phenylmethoxy)-2*H*-1,4-benzoxazin-3(4*H*)-on **2a** werden in 180 L Tetrahydrofuran gelöst und auf-30°C abgekühlt. Es werden 34,63 kg (70,18 mol) (-)-DIP-Chlorid in Hexan 65% innerhalb von 1,5 Stunden zudosiert. Das Reaktionsgemisch wird 1 Stunde nachgerührt und auf 0°C erwärmt. Bei dieser Temperatur werden 11,48 kg (143,54 mol) Natriumhydroxid-Lsg. (50%), mit 36 L Wasser gemischt, zudosiert. Anschließend wird das Zulaufgefäß mit 9 L Wasser gespült. Der pH-Wert am Ende der Zugabe sollte 13 betragen. Es wird auf 20°C erwärmt und 1 Stunde nachgerührt. Eine Mischung aus 4,5 L (42,11 mol) Salzsäure (30%) techn. und 18,6 L Wasser wird solange zudosiert, bis ein pH-Wert von 8,5 erreicht ist. Nach Zugabe von 84 L Ethylacetat wird auf 30°C erwärmt. Nach Phasentrennung wird aus der organischen Phase ein Teil des Lösungsmittels abdestilliert, der Rückstand wird mit 120 L *tert*-Butyl-methylether versetzt, auf 0°C abgekühlt und 1 Stunde nachgerührt. Das Produkt wird isoliert, mit *tert-*Butylmethylether gewaschen und im Vakuum bei 50°C getrocknet. Ausbeute (1a): 8,06 kg (85,0%), Enantiomeren Reinheit laut HPLC: 98,3%.

**Verbindungen der Formel 3b:** 24,68 kg (72,6 mol) Methylmagnesiumchlorid (22%ige Lösung in THF) werden in 35 L Toluol gelöst und auf 16°C abgekühlt. Bei 16 - 22°C wird eine Lösung aus 60,9 mol (4-methoxy-phenyl)-aceton der Formel **4b** und 10 L Toluol zudosiert und bei 22°C 1 Stunde gerührt. Die Reaktionslösung wird zu einer Mischung von 45 L Wasser und 5,22 kg (51,1 mol) Schwefelsäure bei einer Temperatur von 2-17°C zudosiert. Das zweiphasige Gemisch wird gerührt, und die wässrige Phase wird abgetrennt. Die organische Phase wird mit einer Lösung aus 1,00 kg (11,9 mol) Natriumhydrogencarbonat und 11 L Wasser gewaschen. Das Lösungsmittel wird im Vakuum vollständig abdestilliert. Der Rückstand wird in 65,5 L n-Heptan gelöst. Nach Abkühlung auf 2°C wird die Reaktionsmischung 3 Stunden bei dieser Temperatur gerührt. Anschließend wird das Produkt isoliert, mit 17,5 L n-Heptan gewaschen und im Vakuum bei 25°C getrocknet. Ausbeute (3b): 75-80%, Chemische Reinheit laut HPLC: 98.9-99,9%.

**Verbindungen der Formel 2b:** 55,48 mol 1-(4-methoxy-phenyl)-2-methyl-propan-2-ol der Formel **3b** werden in 6,83 kg (166,44 mol) Acetonitril und 13 L Essigsäure vorgelegt und auf 40°C erwärmt. 5,66 kg (55,48 mol) Schwefelsäure werden bei 50 - 55°C zudosiert. Anschließend wird das Gemisch 3 Stunden bei 50°C gerührt. In einem zweiten Reaktor werden 160 L Wasser, 20 L *tert*-Butylmethylether und 21 L Methylcyclohexan auf 10°C gekühlt. Der Inhalt des ersten Reaktors wird in den zweiten Reaktor überführt. Der pH Wert des Reaktorinhalts wird mit ca. 40 I Ammoniaklösung (25%) auf 9,5 eingestellt. Die Suspension wird auf 5°C abgekühlt und 1 Stunde bei dieser Temperatur gerührt. Das Produkt wird abzentrifugiert und mit 30 L Wasser sowie mit einem Gemisch aus 7,5 L *tert-*Butylmethylether und 7,5 L Methylcyclohexan gewaschen. Das feuchte Produkt wird in 25 L Ethanol (96%ig) auf 75°C erwärmt und bei dieser Temperatur mit 30 L Wasser versetzt. Die Lösung wird 15 Minuten bei 85°C gerührt, dann auf 2°C abgekühlt und 1 Stunde bei dieser Temperatur gerührt. Das Produkt wird isoliert, mit einer Mischung aus 5 L Wasser und 5 L Ethanol (96%ig) gewaschen und getrocknet. Ausbeute (2b): 65-71%, Chemische Reinheit laut HPLC: 98.6-99,8%.

**Verbindungen der Formel 1b:** Eine Mischung aus 45,2 mol *N*-[2-(4-methoxy-phenyl)-1,1-dimethyl-ethyl]-acetamid der Formel **2b,** 12,07 kg KOH (180,8 mol), 15 L Ethoxyethanol und 15 L Ethylenglykol wird 12 Stunden auf 150°C erhitzt. Nach Abkühlung auf Raumtemperatur wird das Gemisch mit 61 L Wasser und 31 L Toluol verdünnt. Die Phasen werden getrennt und die organische Phase wird noch einmal mit 30 L Wasser gewaschen. Die organische Phase wird mit 52 L Wasser versetzt. Es wird mit 8,91 kg Salzsäure (90,4 mol) sauer gestellt. Nach Phasentrennung wird die wässrige Produktphase mit 30 L Toluol versetzt und mit 9,04 kg 50%-iger NaOH (113,0 mol) alkalisch gestellt. Nach Phasentrennung wird die organische Produktphase bis zum öligen Rückstand unter Vakuum eingeengt. Ausbeute **(1 b):** 69-75%, Chemische Reinheit laut HPLC: 94-96%

## Patentansprüche

1. Verfahren zur Herstellung der Verbindung der Formel 1, 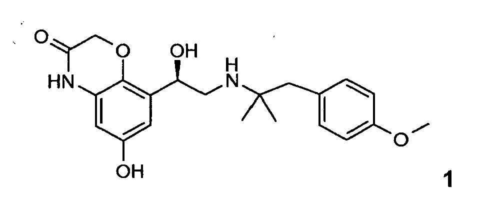 **dadurch gekennzeichnet, dass** eine Verbindung der Formel 2b, 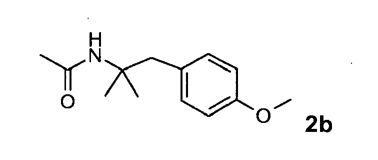 zu einer Verbindung der Formel 1 b, 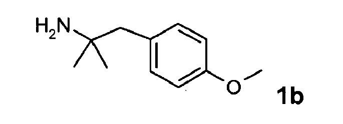 umgesetzt wird und anschließend mit einer Verbindung der Formel **1a,** 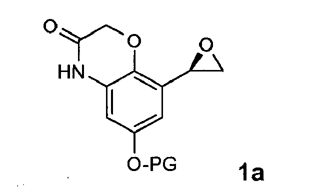 worin PG für eine Schutzgruppe steht, in einem organischen Lösungsmittel zu einer Verbindung der Formel **1c**, 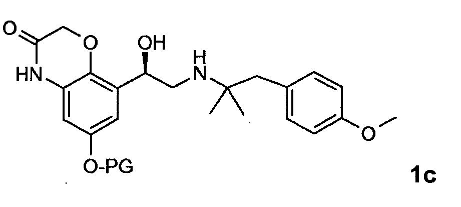 worin PG die oben genannte Bedeutung hat, umgesetzt und aus dieser durch Abspaltung der Schutzgruppe PG die Verbindung der Formel **1** erhalten wird.

2. Verfahren nach Anspruch 1, worin die Verbindung der Formel **1a** durch Umsetzung einer Verbindung der Formel **2a,** 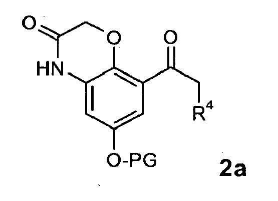 worin PG die in Anspruch 1 genannte Bedeutung hat und R⁴ Halogen bedeutet, mit DIP-Chlorid (Diisopinocampheylchloroboran), hergestellt wird.

3. Verfahren nach Anspruch 2, worin die Umsetzung der Formel **2a** mit DIP-Chlorid (Diisopinocampheylchloroboran) bei einer Temperatur unter 0°C erfolgt.

4. Verfahren nach Anspruch 2 oder 3 worin die Verbindung der Formel **2a** durch Umsetzung einer Verbindung der Formel **3a**, 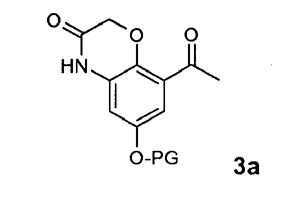 worin PG die in Anspruch 1 genannte Bedeutung hat, hergestellt wird.

5. Verfahren nach Anspruch 4 worin die Verbindung der Formel **3a** durch Umsetzung einer Verbindung der Formel **4a,** 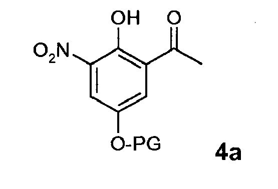 worin PG die in Anspruch 1 genannte Bedeutung hat, hergestellt wird.

6. Verfahren nach Anspruch 5 worin die Verbindung der Formel **4a** durch Umsetzung einer Verbindung der Formel **5a**, 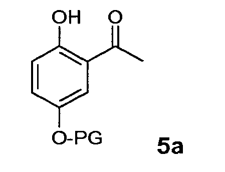 worin PG die in Anspruch 1 genannte Bedeutung hat, hergestellt wird.

7. Verfahren nach Anspruch 6 worin die Verbindung der Formel **5a** durch Umsetzung einer Verbindung der Formel **6a,** 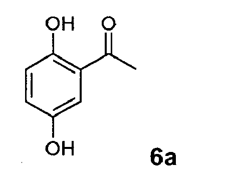 hergestellt wird.

8. Verfahren nach Anspruch 1 worin die Verbindung der Formel 2b durch Umsetzung einer Verbindung der Formel **3b,** 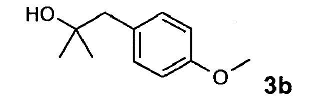 hergestellt wird.

9. Verfahren nach Anspruch 8 worin die Verbindung der Formel 3b durch Umsetzung einer Verbindung der Formel **4b,** 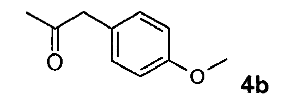 hergestellt wird.

## Claims

1. Process for preparing the compound of formula 1, 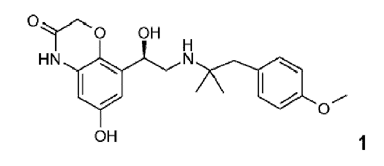 **characterised in that** a compound of formula **2b** 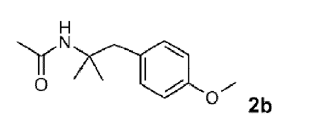 is reacted to form a compound of formula 1 b 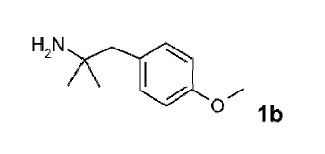 and is then reacted with a compound of formula 1a 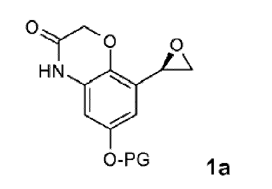 wherein PG denotes a protective group, in an organic solvent, to obtain a compound of formula 1c, 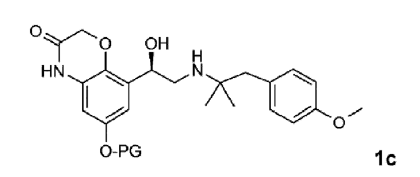 wherein PG has the meaning given above, and the compound of formula 1 is obtained therefrom by cleaving the protective group PG.

2. Process according to claim 1, wherein the compound of formula 1 a is prepared by reacting a compound of formula **2a,** 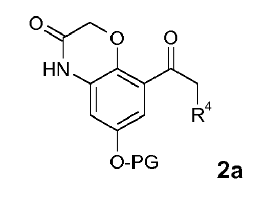 wherein PG has the meaning given in claim 1 and R⁴ denotes halogen, with DIP-chloride (diisopinocampheylchloroborane).

3. Process according to claim 2, wherein the reaction of formula **2a** with DIP-chloride (diisopinocampheylchloroborane) is carried out at a temperature below 0°C.

4. Process according to claim 2 or 3, wherein the compound of formula 2a is prepared by reacting a compound of formula **3a,** 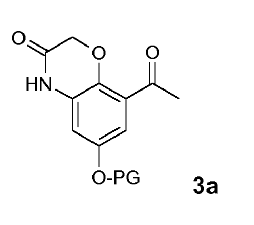 wherein PG has the meaning given in claim 1.

5. Process according to claim **4**, wherein the compound of formula 3a is prepared by reacting a compound of formula **4a**, 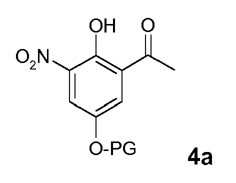 wherein PG has the meaning given in claim 1.

6. Process according to claim 5, wherein the compound of formula **4a** is prepared by reacting a compound of formula 5a, 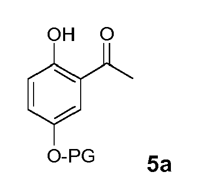 wherein PG has the meaning given in claim 1.

7. Process according to claim 6, wherein the compound of formula **5a** is prepared by reacting a compound of formula **6a,**

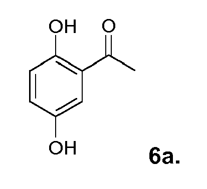

8. Process according to claim 1, wherein the compound of formula **2b** is prepared by reacting a compound of formula **3b**,

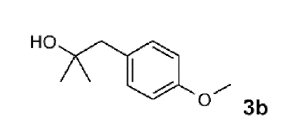

9. Process according to claim 8, wherein the compound of formula **3b** is prepared by reacting a compound of formula **4b,**

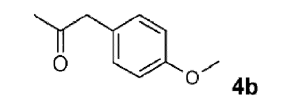

## Revendications

1. Procédé de production du composé de formule 1, 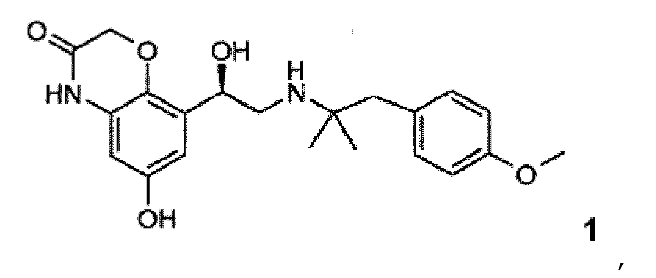 **caractérisé en ce qu'**un composé de formule 2b, 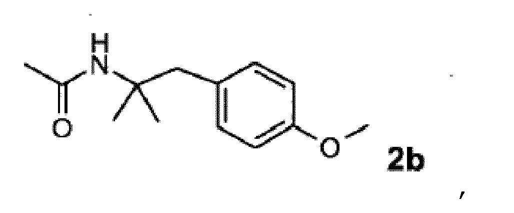 est converti en un composé de formule 1b, 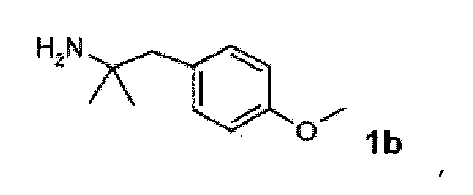 et ensuite est converti avec un composé de formule 1a, 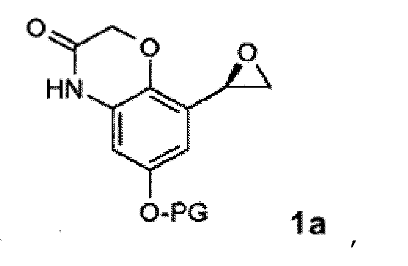 dans laquelle PG représente un groupe protecteur, dans un solvant organique, en un composé de formule 1c, 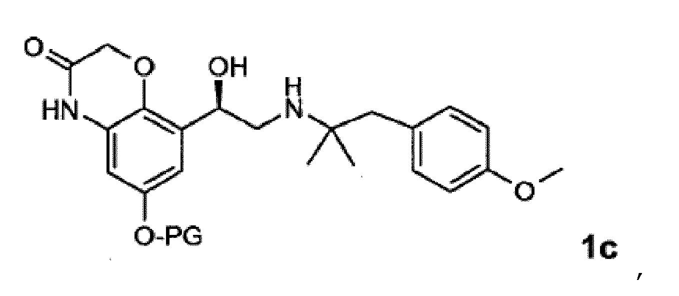 dans laquelle PG a la signification indiquée ci-dessus, et à partir de celui-ci, par clivage du groupe protecteur PG, on obtient le composé de formule 1.

2. Procédé selon la revendication 1, dans lequel le composé de formule 1a est produit par conversion d'un composé de formule 2a, 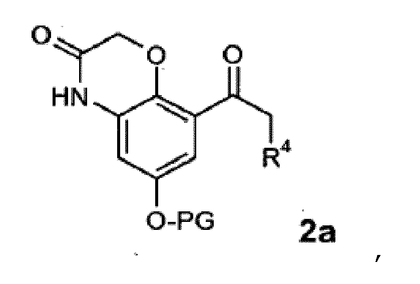 dans laquelle PG a la signification indiquée dans la revendication 1 et R⁴ représente un atome d'halogène, avec du chlorure de DIP (diisopinocamphéylchloroborane).

3. Procédé selon la revendication 2, dans lequel la conversion de la formule 2a s'effectue avec du chlorure de DIP (diisopinocamphéylchloroborane) à une température inférieure à 0 °C.

4. Procédé selon la revendication 2 ou 3, dans lequel le composé de formule 2a est produit par conversion d'un composé de formule 3a, 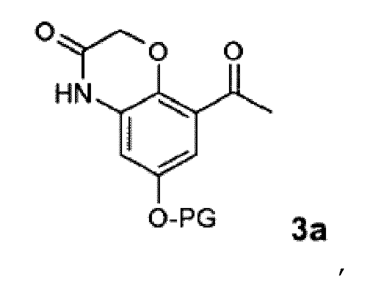 dans laquelle PG a la signification indiquée dans la revendication 1.

5. Procédé selon la revendication 4, dans lequel le composé de formule 3a est produit par conversion d'un composé de formule 4a, 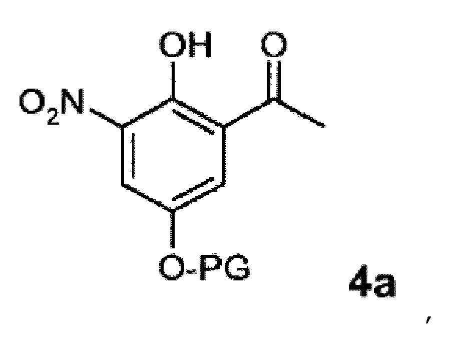 dans laquelle PG a la signification indiquée dans la revendication 1.

6. Procédé selon la revendication 5, dans lequel le composé de formule 4a est produit par conversion d'un composé de formule 5a, 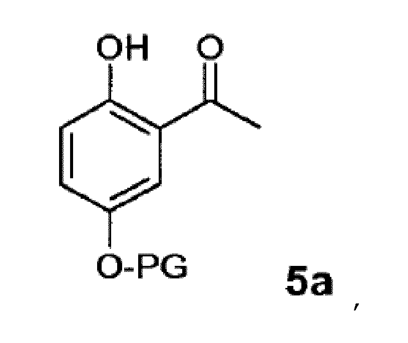 dans laquelle PG a la signification indiquée dans la revendication 1.

7. Procédé selon la revendication 6, dans lequel le composé de formule 5a est produit par conversion d'un composé de formule 6a,

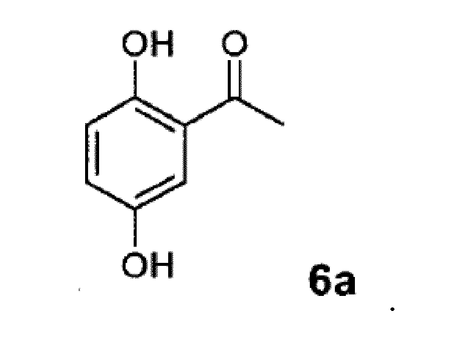

8. Procédé selon la revendication 1, dans lequel le composé de formule 2b est produit par conversion d'un composé de formule 3b,

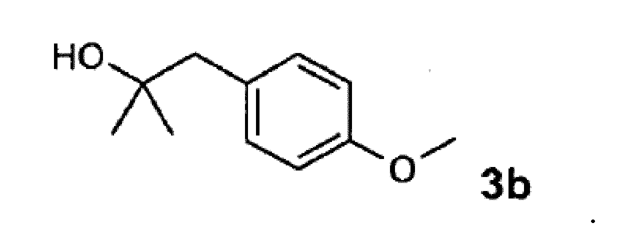

9. Procédé selon la revendication 8, dans lequel le composé de formule 3b est produit par conversion d'un composé de formule 4b,
